# EUROPEAN PATENT SPECIFICATION

(11) **EP 4 180 009 B1**
(45) Date of publication and mention of the grant of the patent: **28.01.2026**
(21) Application number: 20944336.5
(22) Date of filing: 30.10.2020
(51) Int. Cl.: A61F 2/95

(54) **CONVEYING DEVICE AND CONTROL APPARATUS**
FÖRDERVORRICHTUNG UND STEUERUNGSVORRICHTUNG
DISPOSITIF DE TRANSPORT ET APPAREIL DE CONTRÔLE

(30) Priority: 10.07.2020 CN 202010662210
(43) Date of publication of application: 17.05.2023
(73) Proprietor: Shenzhen Chuangxin Medical Technology Co., Ltd., Shenzhen, Guangdong 518118 (CN); Lou, Deda, Shenzhen, Guangdong 518118 (CN)
(72) Inventor: LOU, Deda, Shenzhen, Guangdong 518118 (CN); PENG, Yucheng, Shenzhen, Guangdong 518118 (CN); SU, Chengming, Shenzhen, Guangdong 518118 (CN)
(74) Representative: Viering, Jentschura & Partner mbB Patent- und Rechtsanwälte
(86) International application number: PCT/CN2020/125483
(87) International publication number: WO 2022/007261

(56) References cited:
- CN-A- 103 648 439
- CN-A- 103 655 004
- CN-A- 106 691 648
- CN-A- 108 403 269
- CN-A- 110 786 973
- CN-A- 111 374 814
- CN-A- 111 374 814
- CN-A- 111 772 893
- CN-U- 209 332 380
- US-A1- 2006 265 045
- US-A1- 2011 270 372
- US-A1- 2013 274 859
- US-A1- 2015 305 902
- US-B2- 8 303 546

## Description

### TECHNICAL FIELD

The present application relates to the technical field of medical equipment, and more particularly to a delivery apparatus.

### BACKGROUND

In recent years, some scholars have begun to try to use endovascular exclusion to treat aortic diseases, and related chimney and fenestration techniques have begun to achieve some results, and the results are encouraging. However, due to the particularity of the anatomical structure of the arch of the aorta and the complexity of hemodynamics, the implantation of the vascular stent into the body requires the use of a delivery apparatus for delivery.

Traditional delivery apparatuses generally adopt a hollow inner tube and an outer tube that are sleeved with each other. The hollow portion of the inner tube is convenient for the guide wire to pass through the tube during the operation, and the vascular stent is arranged between the inner tube and the outer tube. Due to the structural defects of the traditional delivery apparatuses, the release process of the vascular stent is too slow, and the operation time is too long, which is not conducive to postoperative recovery, and may even cause postoperative complications.

US Patent application US20060265045A1 provides a method of deploying a prosthesis including engaging a hub assembly of a handle to a threaded outer surface of a slide shaft of the handle; rotating the hub assembly to cause axial translation of the hub assembly and a sheath coupled to the hub assembly to initiate deployment of the prosthesis; disengaging the hub assembly from the threaded outer surface by pivoting a thread tooth of the hub assembly out of threaded engagement with the threaded outer surface; and sliding the hub assembly on the slide shaft to further retract the sheath and complete deployment of the prosthesis.

US Patent application US20150305902A1 provides a delivery system for delivering a prosthesis including a sheath, a slide shaft having a threaded outer surface, and a handle. The handle includes an internal spring assembly for selectively engaging and disengaging the handle with the threaded outer surface of the slide shaft. The internal spring assembly includes at least one spring arm, a head coupled to the spring arm and having a circumferentially rounded threaded inner surface, and a ring slidably disposed over the spring arm. When the ring is in a first longitudinal position, the threaded inner surface of the head is spaced apart from the threaded outer surface of the slide shaft. When the ring is in a second longitudinal position, the threaded inner surface of the head is threadedly engaged with the threaded outer surface of the slide shaft. The handle may include a resilient cover to provide the internal spring assembly with a biased or nominal operational position.

Chinese Patent application CN111374814A provides an adjustable bending sheath tube for conveying a stent into a blood vessel, which comprises: a bendable pipe body; and one end of the traction wire is fixedly connected with one end of the bendable pipe body, the other end of the traction wire penetrates out of the bendable pipe body and penetrates into the bendable pipe body until penetrating out of the other end of the bendable pipe body, and when the other end of the traction wire is pulled, the traction wire drives one end of the bendable pipe body to be close to the other end of the traction wire so that the bendable pipe body is bent.

US Patent application US8303546B2 provides a locking device for preventing movement of an elongated instrument including a tubular base that has an outer surface and an inner surface. At least one lateral opening extends through the tubular base from the outer surface through the inner surface. At least one rocker arm is mounted to the base. Each of the rocker arms has a locking projection that extends radially inwardly aligned with one of the lateral openings of the base. An actuating member is rotatable relative to the base portion between first and second positions. In the first position the actuating member engages the rocker arm to urge the locking projection radially inward through the lateral opening and beyond the inner surface of the tubular base. Movement of the actuating member to the second position causes the locking projection to move radially outward through at least a portion of the lateral opening.

### SUMMARY

Based on this, it is necessary to provide a delivery apparatus and a control device. The control device can control the fast movement and the slow movement of the connecting member, which is beneficial to realize the speed control of the release of the vascular stent. The delivery apparatus adopts the control device, which can realize the speed control of the vascular stent, which is beneficial to shorten the operation time.

The technical solution is as follows:
The present invention provides a delivery apparatus, which includes a control device, and the control device includes a fixing unit and a movable unit; the fixing unit includes a screw rod provided with an accommodating hole and a sliding groove communicating with the accommodating hole, a depth of the accommodating hole and a length of the sliding groove are arranged along an axis direction of the screw rod; and the movable unit includes a connecting member, a first handle, a mating member, and a control member; the connecting member is provided with a first connecting body and a second connecting body fixed to the first connecting body, the first connecting body is arranged in the accommodating hole, and the first connecting body is slidingly engaged with the screw rod, the second connecting body is rotatably connected to the first handle passing through the sliding groove, the first handle is hollow and is sleeved on an outside of the screw rod, and movable relative to the screw rod, the mating member is arranged in the first handle, and the mating member is provided with an internal thread structure engaged with the screw rod, the control member is movably arranged on the first handle, and the first handle drives the mating member to rotate through the control member; when the control member is in a first position, the internal thread structure is separated from the screw rod; and when the control member is in a second position, the internal thread structure is engaged with the screw rod through a thread transmission; and the first connecting body is provided with a connection portion, and the delivery apparatus further includes a delivery tube for delivering a vascular stent, and one end of the delivery tube is fixedly connected with the connecting member through the connection portion;
the movable unit further comprises a sleeve, the sleeve is sleeved on the outside of the screw rod, and the sleeve is provided with a through hole, the mating member is arranged on the sleeve, and the internal thread structure is capable of passing through the through hole to be engaged with the screw rod, the mating member is further provided with a protrusion spaced apart from the internal thread structure in a circumferential direction, the protrusion is arranged in a direction away from the screw rod, and the control member is provided with a pressing body and an extruding body spaced apart from the pressing body;
when the control member is in the first position, the extruding body is offset from the mating member, and the pressing body is press-fitted with the protrusion, such that the internal thread structure is separated from the screw rod; and
when the control member is in the second position, the pressing body is offset from the protrusion, and the extruding body is press-fitted with the mating member, such that the internal thread structure is engaged with the screw rod through a thread transmission, and the first handle is capable of driving the control member and the mating member to rotate.

When the above-mentioned control device is used, the connecting member, the mating member and the control member are integrated on the first handle, and the first connecting body of the connecting member is arranged in the accommodating hole and is directly or indirectly slidingly engaged with the screw rod. At the same time, the first handle is rotatably connected to the connecting member through the second connecting body, such that the first handle can drive the connecting member to move in the axial direction of the screw rod as well as rotate relative to the connecting member. The first connecting body is connected with the delivery tube. Specifically, when applied to a delivery apparatus, when the vascular stent needs to be released quickly, the control member moves to the first position, at this time, the internal thread structure is separated from the screw rod, so that the mating member can move relative to the axial direction of the screw rod, that is, the moving of the first handle can drive the control member, the mating member and the connecting member to move in the axial direction of the screw rod. The connecting member is used to quickly pull the delivery tube so that the vascular stent is released quickly; when the vascular stent is released to a certain extent, fine release control is required, at this time, the control member can be moved to the second position, and the internal thread structure is engaged with the screw rod through a thread transmission, so that the mating member and the screw rod are screwed together. At this time, rotating the first handle can drive the mating member to move along the axis direction of the screw rod, that is, driving the first handle, the control member and the connecting member to move along the axial direction of the screw rod, and the moving distance of the connecting member is controlled by rotating the first handle to realize the fine release of the vascular stent. In this way, the control device can control the fast movement and the slow movement of the connecting member, thereby facilitating the realization of the speed control of the release of the vascular stent.

The further describing of the technical solution are following:
In one of embodiments, the control member is arranged in the sleeve and capable of being elastically reset, and the control member is capable of being automatically reset to the second position under an elastic force; and an inclined surface introduction structure is provided between the extruding body and the mating member.

In one of embodiments, the mating member is arranged on the screw rod and capable of being elastically reset, and the control member is provided with an insertion portion;
when the control member is in the first position, the insertion portion is inserted between the internal thread structure and the screw rod, such that the internal thread structure is separated from the screw rod; and
when the control member is in the second position, the insertion portion is offset from both the internal thread structure and the screw rod, and the mating member is reset under an elastic force, such that the internal thread structure is engaged with the screw rod through a thread transmission.

In one of embodiments, the fixing unit further comprises a second handle, the second handle is fixed on the screw rod, and the first handle is movable relative to the second handle.

In one of embodiments, the control device further includes a bending unit, the bending unit includes a driving member and a flexible traction member, the driving member is movably disposed on the first handle, and the driving member is movable with the first handle, and one end of the flexible traction member is fixed on the driving member.

In one of embodiments, the driving member is rotatable relative to the first handle, and the driving member is provided with a connecting sleeve sleeved on the screw rod and a force applying portion fixed on an outside of the connecting sleeve; one end of the flexible traction member is fixed on the connecting sleeve; or the connecting sleeve is provided with an external thread structure, and the bending unit further includes a third handle fixedly connected with the first handle and a threaded sleeve that is screwed and engaged with the external thread structure, the third handle is hollow, the threaded sleeve is arranged in the third handle and is slidably connected to the third handle, one end of the flexible traction member is fixed on the threaded sleeve.

In one of embodiments, the fixing unit further includes a guide rod, the guide rod is fixed in the screw rod, and the first connecting body is provided with a mating hole that is slidingly fitted with the guide rod.

In one of embodiments, the guide rod is provided with a flow channel, and the fixing unit further includes a guide tube in communicated with the flow channel, and one end of the guide tube is fixed to the screw rod and is communicated with outside.

When the delivery apparatus is in used, when the vascular stent needs to be released quickly, the control member moves to the first position, at this time, the internal thread structure is separated from the screw rod, so that the mating member can move relative to the axial direction of the screw rod, that is, the moving of the first handle can drive the control member, the mating member and the connecting member to move in the axial direction of the screw rod. The connecting member is used to quickly pull the delivery tube so that the vascular stent is released quickly; when the vascular stent is released to a certain extent, fine release control is required, at this time, the control member can be moved to the second position, and the internal thread structure is engaged with the screw rod through a thread transmission, so that the mating member and the screw rod are screwed together. At this time, rotating the first handle can drive the mating member to move along the axis direction of the screw rod, that is, driving the first handle, the control member and the connecting member to move along the axial direction of the screw rod, and the moving distance of the connecting member is controlled by rotating the first handle to realize the fine release of the vascular stent. The delivery apparatus uses the control device, and the speed control of the release of the vascular stent can be realized, which is beneficial to shorten the operation time.

### BRIEF DESCRIPTION OF THE DRAWINGS

Fig. 1 is a schematic view of an embodiment of a delivery apparatus;
Fig. 2 is a use schematic view of the delivery apparatus shown in Fig. 1;
Fig. 3 is a structural explosion view of the delivery apparatus shown in Fig. 1;
Fig. 4 is a partial cross-sectional schematic view of a control device shown in Fig. 1;
Fig. 5 is a cross-sectional schematic view of the control device shown in Fig. 1 from another sight angle;
Fig. 6 is a structural schematic view of the control device shown in Fig. 4 with a part of the first handle is hidden;
Fig. 7 is a structural schematic view of the control device shown in Fig. 6 from another sight angle;
Fig. 8 is a partial cross-sectional schematic view of a bending unit shown in Fig. 1; and
Fig. 9 is a partial enlarged view of the control device shown in Fig. 3.

The reference signs in the drawings are listed:
100-fixing unit; 110-screw rod; 112-accommodating hole; 114-sliding groove; 120-second handle; 130-guide rod; 140-guide tube; 200-movable unit; 210-first handle; 220-connecting member; 222-first connecting body; 202-connection portion; 204-mating hole; 224-second connecting body; 230-mating member; 232-internal thread structure; 234-protrusion; 236-inclined surface introduction structure; 240-control member; 242-pressing body; 244-extruding body; 250-sleeve; 252-through hole; 300-bending unit; 310-driving member; 312-connecting sleeve; 302-external thread structure; 314-force application portion; 320-flexible traction member; 330-third handle; 340-threaded sleeve; 400-delivery tube.

The accompanying drawings constituting a part of the present application are used to provide a further understanding of the present application, the exemplary embodiments and descriptions of the present application are used to explain the present application, and do not constitute an improper limitation of the present application.

In order to explain the technical solutions in the embodiments of the present application more clearly, the following will briefly introduce the accompanying drawings used in the description of the embodiments. Obviously, the accompanying drawings in the following description are only some embodiments of the present application. For those skilled in the art, other drawings can be obtained from these drawings without creative work.

### DETAILED DESCRIPTION

In order to make the objectives, technical solutions, and advantages of the present application clearer, the present application will be further described in detail below with reference to the accompanying drawings and specific embodiments. It should be understood that the specific embodiments described herein are only used to explain the present application, and do not limit the protection scope of the present application.

Unless otherwise defined, all technical and scientific terms used herein have the same meaning as commonly understood by those skilled in the technical field of the present application. The terminology used in the specification of the present application herein is only for the purpose of describing specific embodiments, and is not intended to limit the present application. The term "and/or" as used herein includes any and all combinations of one or more related listed items.

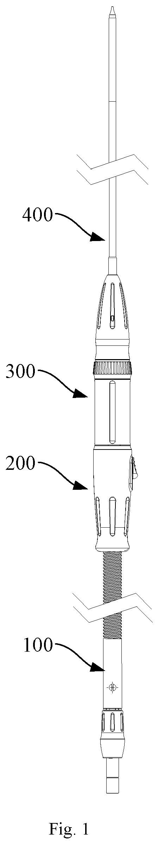

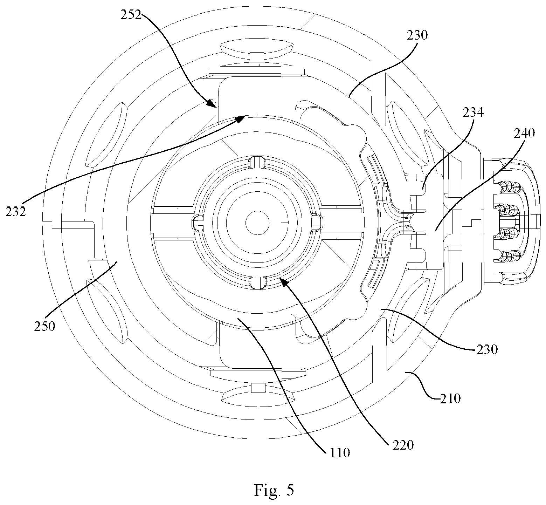

As shown in Figs. 1 and 4 to 7, a control device is provided, which includes a fixing unit 100 and a movable unit 200.

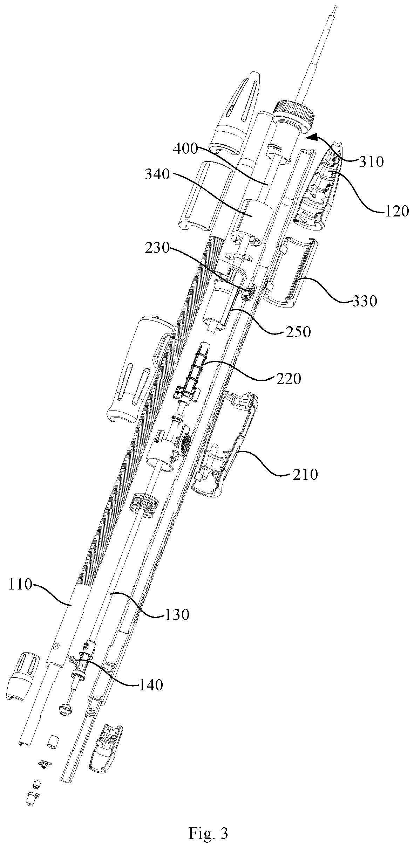

As shown in Figs. 3, 4 and 7, the fixing unit 100 includes a screw rod 100. The screw rod 100 is provided with an accommodating hole 112 and a sliding groove 114 communicating with the accommodating hole 112. The depth of the accommodating hole 112 and the length of the sliding groove 114 are arranged along the axial direction of the screw rod 100.

As shown in Figs. 3 to 7, the movable unit 200 includes a connecting member 220, a first handle 210, a mating member 230, and a control member 240. The connecting member 220 is provided with a first connecting body 222 and a second connecting body 224 fixed to the first connecting body 222. The first connecting body 222 is arranged in the accommodating hole 112, the first connecting body 222 is slidingly engaged with the screw rod 100, the second connecting body 224 passing through the sliding groove 114 and is rotatably connected with the first handle 210, the first handle 210 is hollow and is sleeved in the outer side of the screw rod 100 and can move relative to the screw rod 100.The mating member 230 is arranged in the first handle 210, the mating member 230 is provided with an internal thread structure 232 that fits the screw rod 100, and the control member 240 is movably arranged at the first handle 210. Among them, when the control member 240 is in the first position, the internal thread structure 232 is separated from the screw rod 100; when the control member 240 is in the second position, the internal thread structure 232 is engaged with the screw rod 100 through a thread transmission, and the first handle 210 can drive the control member 240 and the mating member 230 to rotate.

When the above-mentioned control device is used, the connecting member 220, the mating member 230 and the control member 240 are integrated on the first handle 210, and the first connecting body 222 of the connecting member 220 is arranged in the accommodating hole112 and is directly or indirectly slidingly engaged with the screw rod 110.At the same time, the first handle 210 is rotatably connected to the connecting member 220 through the second connecting body 224, such that the first handle 210 can drive the connecting member 220 to move in the axial direction of the screw rod 110 as well as rotate relative to the connecting member 220. The first connecting body 222 is connected with the delivery tube 400. Specifically, when applied to a delivery apparatus, when the vascular stent needs to be released quickly, the control member 240 moves to the first position,at this time, the internal thread structure 232 is separated from the screw rod 110, so that the mating member 230 can move relative to the axial direction of the screw rod 110, that is, the moving of the first handle 210 can drive the control member 240, the mating member 230 and the connecting member 220 to move in the axial direction of the screw rod 110.The connecting member 220 is used to quickly pull the delivery tube 400 so that the vascular stent is released quickly; when the vascular stent is released to a certain extent, fine release control is required,at this time, the control member 240 can be moved to the second position, and the internal thread structure 232 is engaged with the screw rod 110 through a thread transmission, so that the mating member 230 and the screw rod 110 are screwed together. At this time, rotating the first handle 210 can drive the mating member 230 to move along the axis direction of the screw rod 110, that is, driving the first handle 210, the control member 240 and the connecting member 220 to move along the axial direction of the screw rod 110, and the moving distance of the connecting member 220 is controlled by rotating the first handle 210 to realize the fine release of the vascular stent. In this way, the control device can control the fast movement and the slow movement of the connecting member 220, thereby facilitating the realization of the speed control of the release of the vascular stent.

It should be noted that there can be multiple forms of fitting between the "control member 240" and the "mating member 230", as long as the separation and the transmission between the internal thread structure 232 and the screw rod 110 can be c switched.

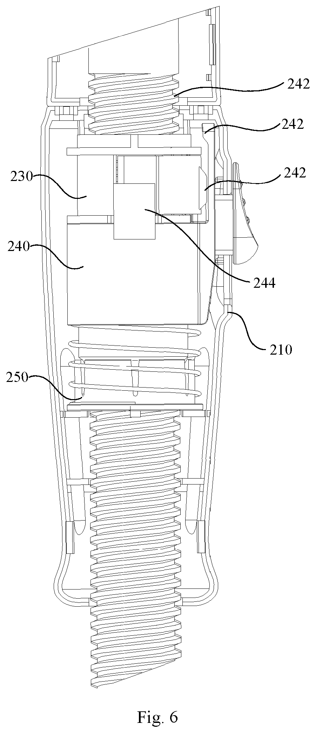

Specifically in this embodiment, as shown in Figs. 6, 7 and 9, the movable unit 200 further includes a sleeve 250, which is sleeved on the outside of the screw rod 100, the sleeve 250 is provided with a through hole 252, and the mating member 230 is arranged on the sleeve 250, and the internal thread structure 232 can pass through the through hole 252 to fit with the screw rod 100. The mating member 230 is further provided with a protrusion 234 spaced from the internal thread structure 232 in the circumferential direction. The protrusion 234 is arranged in a direction f away from the screw rod 110, the control member 240 is provided with a pressing body and an extruding body arranged apart from the pressing body. When the control member 240 is in the first position, the extruding body is offset from the mating member 230, and the pressing body is press-fitted with the protrusion 234, so that the internal thread structure 232 is separated from the screw rod 100; when the control member 240 is in the second position, the pressing body is offset from the extension 234, the extruding body is press-fitted with the mating member, so that the internal thread structure 232 is engaged with the screw rod 100 through a thread transmission.

In this way, the mating member 230 is arranged on the sleeve 250 to form a seesaw structure. When the control member 240 moves to the first position, the extruding body is offset from the mating member 230, and the pressing body is press-fitted with the protrusion 234, so that the internal thread structure 232 is raised and separated from the screw rod 100; when the control member 240 moves to the second position, the pressing body is offset from the protrusion 234, the extruding body is press-fitted with the mating member, and the internal thread structure 232 is reset, so that the internal thread structure 232 is engaged with the screw rod 100 through a thread transmission. In this process, only by moving the control member 240 along the axial direction of the screw rod 100 can switch between the first position and the second position, and switch the separation and transmission cooperation between the internal thread structure 232 and the screw rod 100.

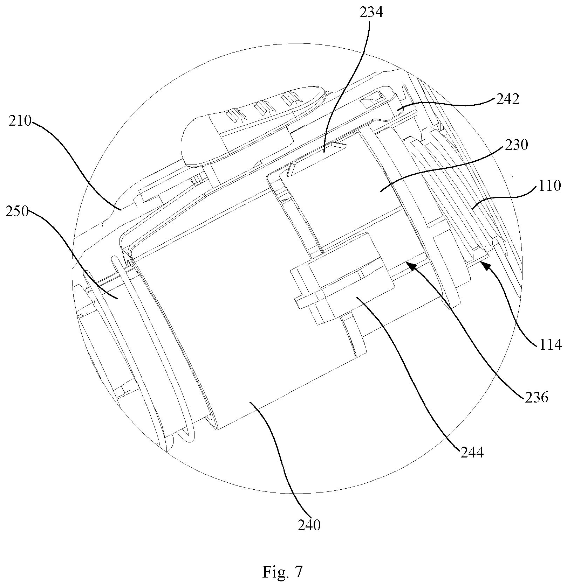

Based on the above embodiment, as shown in Figs. 6 and 7, in one embodiment, the control member 240 can be elastically reset on the sleeve 250, and under the elastic force, the control member 240 can be automatically reset to the second position. There is an inclined surface introduction structure 236 is provided between the extruding body and the mating member. In this way, under the elastic force, the control member 240 can automatically reset to the second position, so that the mating member 230 and the screw rod 100 are screwed together, and then the first handle 210 can be rotated to drive the connecting member 220 slowly along the axial direction of the screw rod 100, the movement facilitates the fine control of the release of the vascular stent.At the same time, by arranging the inclined surface introduction structure 236, it is convenient to press-fit the extruding body to the mating member, so that the internal thread structure 232 is engaged with the screw rod 100 through a thread transmission. The inclined surface introduction structure 236 can be arranged on the extruding body or/and the mating member to play the role of first introduction and gradual extrusion.

Of course, in another embodiment, the control member 240 includes a first pressing portion (not shown) and a second pressing portion (not shown); when the first pressing portion is pressed down to the first position, the second pressing portion is not at the second position, the pressing body is press-fitted with the protrusion 234, so that the internal thread structure 232 is raised and separated from the screw rod 100; when the second pressing portion is pressed down to the second position, the first pressing portion is not at the first position, the pressing body is offset from the protrusion 234, and the extruding body is press-fitted the internal thread structure 232, so that the internal thread structure 232 is reset, and the internal thread structure 232 and the screw rod 100 are screwed together. In this way, the control member 240 can also be used to switch between the two positions by using the pressing principle.

The first pressing portion and the second pressing portion can be realized by using existing technologies such as indexing pins.

In further embodiment, the mating member 230 can be elastically reset and arranged on the screw rod 100, and the control member 240 is provided with an insertion portion (not shown). When the control member 240 is in the first position, the insertion portion is inserted between the internal thread structure 232 and the screw rod 100,so that the internal thread structure 232 is separated from the screw rod 100; when the control member is in the second position, the insertion portion is offset from both the internal thread structure 232 and the screw rod 100, and the mating member 230 is reset under the elastic force, so that the internal thread structure 232 is engaged with the screw rod 100 through a thread transmission. In this way, by automatically resetting the insertion portion and the mating member 230, it is also possible to switch the separation and the transmission cooperation between the internal thread structure 232 and the screw rod 100.

The insertion portion can be movably inserted between the internal thread structure 232 and the screw rod 100, or can be inserted between the internal thread structure 232 and the screw rod 100 by pressing, which can be selected according to actual needs.

On the basis of any of the foregoing embodiments, as shown in Fig. 3, in one embodiment, the fixing unit 100 further includes a second handle 120, the second handle 120 is fixed on the screw rod 100, and the first handle 210 can move relative to the second handle 120. In this way, by arranging the second handle 120, it is convenient for the operator to grab the second handle 120 to perform related operations on the first handle 210, and realize the movement and rotation of the first handle 210.

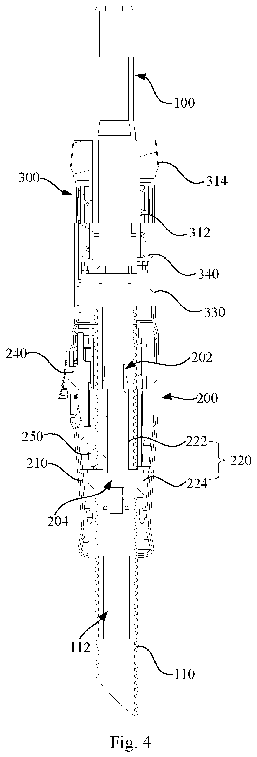

Based on any of the foregoing embodiments, as shown in Figs. 4, 8 and 9, in one embodiment, the control device further includes a bending unit 300, and the bending unit 300 includes a driving member 310 and a flexible traction member 320, and the driving member 310 is movably arranged on the first handle 210, and the driving member 310 can move with the first handle 210, and one end of the flexible traction member 320 is fixed on the driving member 310. In this way, the other end of the flexible traction member 320 is fixedly connected to the free end of the delivery tube 400. When the delivery tube 400 enters the arch portion of the aorta, such that the driving member 310 is moved driving the flexible traction member 320 to pull the delivery tube 400 to bend, so that the delivery tube 400 enables the vascular stent to pass through the arch portion of the larger aorta smoothly and safely, overcomes the problem of easy damage to the arch portion of the aortic during the delivery of the vascular stent, and reduces the risk of surgery.

Further, in one embodiment, the driving member 310 can rotate relative to the first handle 210, and the driving member 310 is provided with a connecting sleeve 312 sleeved on the screw rod 100 and a force applying portion 314 fixed on the outside of the connecting sleeve 312, one end of the flexible traction member 320 is fixed on the connecting sleeve 312.In this way, when it is necessary to perform bending control, only need to rotate the force application portion 314 making the connecting sleeve 312 to rotate, which in turn drives the flexible traction member 320 to shrink and wrap around the connecting sleeve 312, so that the delivery tube 400 bends so as to smoothly pass through the curved portion of the blood vessel.

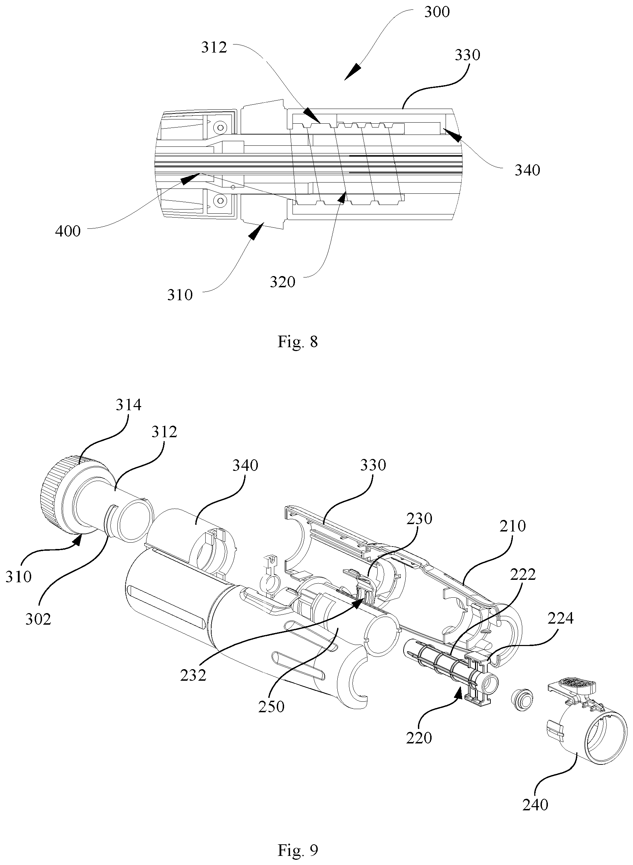

Alternatively, in another embodiment, as shown in Figs. 8 and 9, the driving member 310 can rotate relative to the first handle 210, and the driving member 310 is provided with a connecting sleeve 312 sleeved on the screw rod 100 and a force application portion 314 fixed on the outer side of the connecting sleeve 312, the connecting sleeve 312 is provided with an external thread structure 302, and the bending unit 300 further includes a third handle 330 fixedly connected to the first handle 210, and a threaded sleeve 340 that is screwed to the external thread structure 302.The third handle 330 is hollow, and the threaded sleeve 340 is arranged in the third handle 330 and is slidably connected to the third handle 330, one end of the flexible traction member 320 is fixed on the threaded sleeve 340.In this way, when the bending control is required, only the force application portion 314 needs to be rotated making the connecting sleeve 312 to rotate, which in turn drives the threaded sleeve 340 to move along the axis of the connecting sleeve 312, and drives the flexible traction member 320 to be tightened, such that the delivery tube 400 is bent, so as to smoothly pass through the curved portion of the blood vessel.

It should be noted that the flexible traction member 320 is a traction rope or a traction chain.

Specifically, in the embodiment, the flexible traction member 320 is a traction rope, which is beneficial to save space, so that the volume of the delivery device can be smaller, especially the delivery tube 400 has a smaller volume, which is suitable for minimally invasive surgery.

On the basis of any of the above embodiments, as shown in Fig. 3, in one embodiment, the fixing unit 100 further includes a guide rod 130, the guide rod 130 is fixed in the screw rod 100, and the first connecting body 222 is provided with a mating hole 204 for sliding fit with the guide rod 130. In this way, through the sliding fit of the guide rod 130 and the mating hole 204, the connecting member 220 can slide relative to the screw rod 100, so that the connecting member 220 moves more smoothly.

Further, in one embodiment, the guide rod 130 is provided with a flow channel (not shown), and the fixing unit 100 further includes a guide tube 140 that communicates with the flow channel. One end of the guide tube 140 is fixed on the screw rod 100 and communicated to the outside. In this way, the required reagent can be injected into the flow channel through the guide tube 140, and the reagent flows into the delivery tube 400 through the guide tube 140, the flow channel, and the mating hole 204, and flows out of the vascular stent, so that treatment can be better.

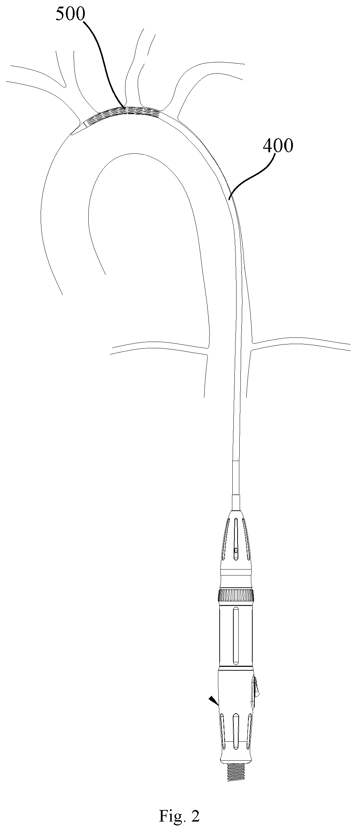

As shown in Figs. 1 to 3, in one embodiment, a delivery apparatus is further provided, which includes the control device in any of the above embodiments, the first connecting body 222 is provided with a connection portion 202, and further includes a delivery tube 400, and one end of the delivery tube 400 is fixedly connected to the connecting member 220 through the connection portion 202.

When the delivery apparatus is in used, when the vascular stent needs to be released quickly, the control member 240 moves to the first position,at this time, the internal thread structure 232 is separated from the screw rod 110, so that the mating member 230 can move relative to the axial direction of the screw rod 110, that is, the moving of the first handle 210 can drive the control member 240, the mating member 230 and the connecting member 220 to move in the axial direction of the screw rod 110.The connecting member 220 is used to quickly pull the delivery tube 400 so that the vascular stent is released quickly; when the vascular stent is released to a certain extent, fine release control is required, at this time, the control member 240 can be moved to the second position, and the internal thread structure 232 is engaged with the screw rod 110 through a thread transmission, so that the mating member 230 and the screw rod 110 are screwed together. At this time, rotating the first handle 210 can drive the mating member 230 to move along the axis direction of the screw rod 110, that is, driving the first handle 210, the control member 240 and the connecting member 220 to move along the axial direction of the screw rod 110, and the moving distance of the connecting member 220 is controlled by rotating the first handle 210 to realize the fine release of the vascular stent. The delivery apparatus adopts the control device, which can realize the speed control of the vascular stent, which is beneficial to shorten the operation time.

Specifically, the delivery apparatus is fitted with a vascular stent and can be applied to the treatment of Stanford Type A aortic dissection. Stanford Type A acute aortic dissection type A (referred to as AADA) is a catastrophic disease that severely endangers the patient's life and safety. It is characterized by sudden onset, rapid disease progression, and high mortality.

It should be noted that "body" and "portion" can be part of the corresponding "component", that is, "body", "portion" and the "other part of the component" are integrally formed and manufactured; or they can be separated as an independent component with the "other part of the component", that is, the "body" and "portion" can be manufactured independently, and then combined with the "other part of the component" into a whole. The expressions of the above-mentioned "body" and "portion" in the present application are only one example. For the convenience of reading, it is not a limitation on the scope of protection of the present application. As long as the above features are included and the functions are the same, it should be understood as the technical solution equivalent to the present application.

It should be noted that the components included in the "units", "components", "mechanisms" and "devices" of the present application can also be flexibly combined, and modular production can be carried out according to actual needs to facilitate modular assembly. The division of the above-mentioned components in the present application is only one of the embodiments. For the convenience of reading, it is not a limitation of the protection scope of the present application. As long as the above-mentioned components are included and have the same function, it should be understood that it is an equivalent technical solution of the present application.

In the description of the present application, it should be understood that the terms "center", "longitudinal", "transverse", "length", "width", "thickness", "upper", "lower", "front", " back", "left", "right", "vertical", "horizontal", "top", "bottom", "inner" , "outer" , "clockwise", "counterclockwise", "axial" , "radial", "circumferential", etc. indicate the orientation or positional relationship based on the orientation or positional relationship shown in the drawings, and are only for the convenience of describing the present application and simplifying the description, rather than indicating or implying the pointed device or the element must have a specific orientation, be constructed and operated in a specific orientation, and therefore cannot be understood as a limitation of the present application.

In addition, the terms "first" and "second" are only used for descriptive purposes, and cannot be understood as indicating or implying relative importance or implicitly indicating the number of indicated technical features. Therefore, the features defined with "first" and "second" may explicitly or implicitly include at least one of the features. In the description of the present application, "plurality" means at least two, such as two, three, etc., unless otherwise specifically defined.

In the present application, unless otherwise clearly specified and limited, the terms "installed", "connected", "connecting", "fixed" and other terms should be understood in a broad sense, for example, it can be a fixed connection or a detachable connection, or integrated; it can be mechanically connected or electrically connected; it can be directly connected or indirectly connected through an intermediary, it can be the internal communication of two components or the interaction relationship between two components, unless otherwise specified limitation. For those skilled in the art, the specific meaning of the above-mentioned terms in the present application can be understood according to specific circumstances.

In the present application, unless expressly stipulated and defined otherwise, the first feature "on" or "under" the second feature may be in direct contact with the first and second features, or the first and second features may be indirectly contacted through an intermediary. Moreover, the "above" or "on" of the first feature on the second feature may mean that the first feature is directly above or obliquely above the second feature, or it simply means that the level of the first feature is higher than the second feature. The first feature "below" or "under" the second feature can mean that the first feature is directly below or obliquely below the second feature, or it simply means that the level of the first feature is smaller than the second feature.

It should be noted that when an element is referred to as being "fixed to", "arranged on", "disposed on" or "installed on" another element, it can be directly on the other element or there may also be a centered element.. When an element is considered to be "connected" to another element, it can be directly connected to the other element or an intermediate element may be present at the same time. Further, when one element is regarded as a "fixed transmission connection" and another element, the two can be fixed in a detachable connection or non-detachable connection, which can realize power transmission, such as sleeved connection and engagement, one-piece molding fixing, welding, etc., which can be realized in the prior art, and it is no longer burdensome here. When a component and another component are perpendicular or approximately perpendicular to each other, it means that the ideal state of the two is perpendicular, but due to the influence of manufacturing and assembly, there may be a certain vertical error. The terms "vertical", "horizontal", "left", "right" and similar expressions used herein are for illustrative purposes only, and do not mean that they are the only embodiments.

## Claims

1. A delivery apparatus, comprising: a control device, wherein the control device comprises:
a fixing unit (100), comprising a screw rod (110) provided with an accommodating hole (112) and a sliding groove (114) communicating with the accommodating hole (112), wherein a depth of the accommodating hole (112) and a length of the sliding groove (114) are arranged along an axis direction of the screw rod (110); and
a movable unit (200), comprising a connecting member (220), a first handle (210), a mating member (230), and a control member (240); wherein the connecting member (220) is provided with a first connecting body (222) and a second connecting body (224) fixed to the first connecting body (222), the first connecting body (222) is arranged in the accommodating hole (112), and the first connecting body (222) is slidingly engaged with the screw rod (110), the second connecting body (224) is rotatably connected to the first handle (210) passing through the sliding groove (114), the first handle (210) is hollow and is sleeved on an outside of the screw rod (110), and movable relative to the screw rod (110), the mating member (230) is arranged in the first handle (210), and the mating member (230) is provided with an internal thread structure (232) engaged with the screw rod (110), the control member (240) is movably arranged on the first handle (210), and the first handle (210) drives the mating member (230) to rotate through the control member (240);
wherein when the control member (240) is in a first position, the internal thread structure (232) is separated from the screw rod (110);
when the control member (240) is in a second position, the internal thread structure (232) is engaged with the screw rod (110) through a thread transmission;
wherein the first connecting body (222) is provided with a connection portion (202), and the delivery apparatus further comprises a delivery tube (400) for delivering a vascular stent, and one end of the delivery tube (400) is fixedly connected with the connecting member (220) through the connection portion (202);
wherein the movable unit (200) further comprises a sleeve (250), the sleeve is sleeved on the outside of the screw rod (110), and the sleeve (250) is provided with a through hole (252), the mating member (230) is arranged on the sleeve (250), and the internal thread structure (232) is capable of passing through the through hole (252) to be engaged with the screw rod (110), the mating member (230) is further provided with a protrusion (234) spaced apart from the internal thread structure (232) in a circumferential direction, the protrusion (234) is arranged in a direction away from the screw rod (110), and the control member (240) is provided with a pressing body (242) and an extruding body (244) spaced apart from the pressing body (242);
when the control member (240) is in the first position, the extruding body (244) is offset from the mating member (230), and the pressing body (242) is press-fitted with the protrusion (234), such that the internal thread structure (232) is separated from the screw rod (110); and
when the control member (240) is in the second position, the pressing body (242) is offset from the protrusion (234), and the extruding body (244) is press-fitted with the mating member (230), such that the internal thread structure (232) is engaged with the screw rod (110) through a thread transmission, and the first handle (210) is capable of driving the control member (240) and the mating member (230) to rotate.

2. The delivery apparatus according to claim 1, wherein the control member (240) is arranged in the sleeve (250) and capable of being elastically reset, the control member (240) is capable of being automatically reset to the second position under an elastic force; and an inclined surface introduction structure (236) is provided between the extruding body (244) and the mating member (230).

3. The delivery apparatus according to claim 1, wherein the mating member (230) is arranged on the screw rod (110) and capable of being elastically reset, the control member (240) is provided with an insertion portion;
when the control member (240) is in the first position, the insertion portion is inserted between the internal thread structure (232) and the screw rod (110), such that the internal thread structure (232) is separated from the screw rod (110); and
when the control member (240) is in the second position, the insertion portion is offset from both the internal thread structure (232) and the screw rod (110), and the mating member (230) is reset under an elastic force, such that the internal thread structure (232) is engaged with the screw rod (110) through a thread transmission.

4. The delivery apparatus according to claim 1, wherein the fixing unit (100) further comprises a second handle (120), the second handle (120) is fixed on the screw rod (110), and the first handle (210) is movable relative to the second handle (120).

5. The delivery apparatus according to claim 1, wherein the control device further comprises a bending unit (300), the bending unit (300) comprises a driving member (310) and a flexible traction member (320), the driving member (310) is movably disposed on the first handle (210), and the driving member (310) is movable with the first handle (210), and one end of the flexible traction member (320) is fixed on the driving member (310).

6. The delivery apparatus according to claim 5, wherein the driving member (310) is rotatable relative to the first handle (210), and the driving member (310) is provided with a connecting sleeve (312) sleeved on the screw rod (110) and a force applying portion (314) fixed on an outside of the connecting sleeve (312); one end of the flexible traction member (320) is fixed on the connecting sleeve (312); or the connecting sleeve (312) is provided with an external thread structure (302), and the bending unit (300) further comprises a third handle (330) fixedly connected with the first handle (210) and a threaded sleeve (340) that is screwed and engaged with the external thread structure (302), the third handle (330) is hollow, the threaded sleeve (340) is arranged in the third handle (330) and is slidably connected to the third handle (330), one end of the flexible traction member (320) is fixed on the threaded sleeve (340).

7. The delivery apparatus according to any one of claims 1 to 6, wherein the fixing unit (100) further comprises a guide rod (130), the guide rod (130) is fixed in the screw rod (110), and the first connecting body (222) is provided with a mating hole (204) that is slidingly fitted with the guide rod (130).

8. The delivery apparatus according to claim 7, wherein the guide rod (130) is provided with a flow channel, and the fixing unit (100) further comprises a guide tube (140) in communicated with the flow channel, and one end of the guide tube (140) is fixed to the screw rod (110) and is communicated with outside.

## Patentansprüche

1. Zufuhrvorrichtung, umfassend: eine Steuerungsvorrichtung, wobei die Steuerungsvorrichtung umfasst:
eine Befestigungseinheit (100), die eine Gewindestange (110) umfasst, die mit einem Aufnahmeloch (112) und einer Gleitnut (114) versehen ist, die mit dem Aufnahmeloch (112) in Verbindung steht, wobei eine Tiefe des Aufnahmelochs (112) und eine Länge der Gleitnut (114) entlang einer Achsrichtung der Gewindestange (110) angeordnet sind; und
eine bewegliche Einheit (200), die ein Verbindungselement (220), einen ersten Griff (210), ein Passelement (230) und ein Steuerungselement (240) umfasst; wobei das Verbindungselement (220) mit einem ersten Verbindungskörper (222) und einem zweiten Verbindungskörper (224), der an dem ersten Verbindungskörper (222) befestigt ist, versehen ist,
wobei der erste Verbindungskörper (222) in dem Aufnahmeloch (112) angeordnet ist und der erste Verbindungskörper (222) mit der Gewindestange (110) im Gleiteingriff ist, wobei der zweite Verbindungskörper (224) drehbar mit dem ersten Griff (210) verbunden ist, der durch die Gleitnut (114) hindurchgeht, wobei der erste Griff (210) hohl ist und an einer Außenseite der Gewindestange (110) mit einer Hülle versehen und bezogen auf die Gewindestange (110) beweglich ist,
wobei das Passelement (230) in dem ersten Griff (210) angeordnet ist und das Passelement (230) mit einer Innengewindestruktur (232) versehen ist, die mit der Gewindestange (110) im Eingriff ist, wobei das Steuerungselement (240) beweglich an dem ersten Griff (210) angeordnet ist und der erste Griff (210) das Passelement (230) antreibt, um durch das Steuerungselement (240) zu drehen;
wobei, wenn das Steuerungselement (240) in einer ersten Position ist, die innere Gewindestruktur (232) von der Gewindestange (110) getrennt ist;
wobei, wenn das Steuerungselement (240) in einer zweiten Position ist, die innere Gewindestruktur (232) durch einen Gewindeadapter mit der Gewindestange (110) im Eingriff ist;
wobei der erste Verbindungskörper (222) mit einem Verbindungsabschnitt (202) versehen ist und die Zufuhrvorrichtung ferner ein Zufuhrrohr (400) zum Zuführen eines vaskulären Stents umfasst, und wobei ein Ende des Zufuhrrohrs (400) durch den Verbindungsabschnitt (202) fest mit dem Verbindungselement (220) verbunden ist;
wobei die bewegliche Einheit (200) ferner eine Hülse (250) umfasst, wobei die Hülse auf die Außenseite der Gewindestange (110) geschoben ist und die Hülse (250) mit einem Durchgangsloch (252) versehen ist, wobei das Passelement (230) an der Hülse (250) angeordnet ist und die innere Gewindestruktur (232) fähig ist, durch das Durchgangsloch (252) hindurch zu gehen, um mit der Gewindestange (110) in Eingriff gebracht zu werden,
wobei das Passelement (230) ferner mit einem Vorsprung (234) versehen ist, der von der inneren Gewindestruktur (232) in einer Umfangsrichtung beabstandet ist, wobei der Vorsprung (234) in einer Richtung von der Gewindestange (110) weg angeordnet ist und das Steuerungselement (240) mit einem Druckkörper (242) und einem Extrusionskörper (244) versehen ist, der von dem Druckkörper (242) beabstandet ist;
wenn das Steuerungselement (240) in der ersten Position ist, ist der Extrusionskörper (244) von dem Passelement (230) versetzt und der Druckkörper (242) ist mit dem Vorsprung (234) in Presspassung, so dass die innere Gewindestruktur (232) von der Gewindestange (110) getrennt ist; und
wenn das Steuerungselement (240) in der zweiten Position ist, ist der Druckkörper (242) von dem Vorsprung (234) versetzt und der Extrusionskörper (244) ist mit dem Passelement (230) in Presspassung, so dass die innere Gewindestruktur (232) durch einen Gewindeadapter mit der Gewindestange (110) im Eingriff ist, und ist der erste Griff (210) fähig, das Steuerungselement (240) und das Passelement (230) anzutreiben, um sich zu drehen.

2. Zufuhrvorrichtung nach Anspruch 1, wobei das Steuerungselement (240) in der Hülse (250) angeordnet und fähig ist, elastisch zurückgesetzt zu werden, wobei das Steuerungselement (240) fähig ist, unter einer elastischen Kraft automatisch in die zweite Position zurückgesetzt zu werden; und eine geneigte Oberflächeneinführstruktur (236) zwischen dem Extrusionskörper (244) und dem Passelement (230) versehen ist.

3. Zufuhrvorrichtung nach Anspruch 1, wobei das Passelement (230) an der Gewindestange (110) angeordnet und fähig ist, elastisch zurückgesetzt zu werden, wobei das Steuerungselement (240) mit einem Einführabschnitt versehen ist;
wobei, wenn das Steuerungselement (240) in der ersten Position ist, der Einführabschnitt zwischen der inneren Gewindestruktur (232) und der Gewindestange (110) eingeführt ist, so dass die innere Gewindestruktur (232) von der Gewindestange (110) getrennt ist; und
wobei, wenn das Steuerungselement (240) in der zweiten Position ist, der Einführabschnitt sowohl von der inneren Gewindestruktur (232) als auch der Gewindestange (110) versetzt ist, und das Passelement (230) unter einer elastischen Kraft zurückgesetzt wird, so dass die innere Gewindestruktur (232) durch einen Gewindeadapter von der Gewindestange (110) getrennt ist.

4. Zufuhrvorrichtung nach Anspruch 1, wobei die Befestigungseinheit (100) ferner einen zweiten Griff (120) umfasst, wobei der zweite Griff (120) an der Gewindestange (110) befestigt ist, und wobei der erste Griff (210) bezogen auf den zweiten Griff (120) beweglich ist.

5. Zufuhrvorrichtung nach Anspruch 1, wobei die Steuerungsvorrichtung ferner eine Biegeeinheit (300) umfasst, wobei die Biegeeinheit (300) ein Antriebselement (310) und ein flexibles Zugelement (320) umfasst, wobei das Antriebselement (310) beweglich an dem ersten Griff (210) angeordnet ist, und das Antriebselement (310) mit dem ersten Griff (210) beweglich ist und ein Ende des flexiblen Zugelements (320) an dem Antriebselement (310) befestigt ist.

6. Zufuhrvorrichtung nach Anspruch 5, wobei das Antriebselement (310) bezogen auf den ersten Griff (210) drehbar und das Antriebselement (310) mit einer Verbindungshülse (312) versehen ist, die auf die Gewindestange (110) aufgeschoben ist, und ein Kraftaufbringabschnitt (314) an einer Außenseite der Verbindungshülse (312) befestigt ist; ein Ende des flexiblen Zugelements (320) an der Verbindunghülse (312) befestigt ist; oder die Verbindungshülse (312) mit einer äußeren Gewindestruktur (302) versehen ist, und die Biegeeinheit (300) ferner einen dritten Griff (330) umfasst, der fest mit dem ersten Griff (210) verbunden ist, und eine Gewindehülse (340), die mit der äußeren Gewindestruktur (302) verschraubt und damit im Eingriff ist, der dritte Griff (330) hohl ist, die Gewindehülse (340) in dem dritten Griff (330) angeordnet und verschiebbar mit dem dritten Griff (330) verbunden ist, ein Ende des flexiblen Zugelements (320) an der Gewindehülse (340) befestigt ist.

7. Zufuhrvorrichtung nach einem der Ansprüche 1 bis 6, wobei die Befestigungseinheit (100) ferner eine Führungsstange (130) umfasst, wobei die Führungsstange (130) in der Gewindestange (110) befestigt ist und der erste Verbindungskörper (222) mit einem Passloch (204) versehen ist, das mit der Führungsstange (130) verschiebbar ist.

8. Zufuhrvorrichtung nach Anspruch 7, wobei die Führungsstange (130) mit einem Fließkanal versehen ist, und die Befestigungseinheit (100) ferner ein Führungsrohr (140) in Verbindung mit dem Fließkanal umfasst, und ein Ende des Führungsrohrs (140) an der Gewindestange (110) befestigt ist und mit der Außenseite in Verbindung steht.

## Revendications

1. Appareil de pose, comprenant : un dispositif de commande, le dispositif de commande comprenant :
une unité de fixation (100), comprenant une tige de vis (110) pourvue d'un trou de réception (112) et d'une rainure de glissement (114) communiquant avec le trou de réception (112), dans laquelle la profondeur du trou de réception (112) et la longueur de la rainure de glissement (114) sont disposées le long d'une direction d'axe de la tige de vis (110) ; et
une unité mobile (200), comprenant un élément de liaison (220), une première poignée (210), un élément d'accouplement (230), et un élément de commande (240) ;
dans laquelle l'élément de liaison (220) est pourvu d'un premier corps de liaison (222) et d'un second corps de liaison (224) fixé au premier corps de liaison (222), le premier corps de liaison (222) est disposé dans le trou de réception (112), et le premier corps de liaison (222) est en prise de manière coulissante avec la tige de vis (110), le second corps de liaison (224) est relié de manière rotative à la première poignée (210) par le biais de la rainure de glissement (114), la première poignée (210) est creuse et est manchonnée sur l'extérieur de la tige de vis (110), et mobile par rapport à la tige de vis (110), l'élément d'accouplement (230) est disposé dans la première poignée (210), et l'élément d'accouplement (230) est pourvu d'une structure filetée interne (232) en prise avec la tige de vis (110), l'élément de commande (240) est disposé de manière mobile sur la première poignée (210), et la première poignée (210) entraîne la rotation de l'élément d'accouplement (230) par le biais de l'élément de commande (240) ;
dans lequel lorsque l'élément de commande (240) est dans une première position, la structure filetée interne (232) est séparée de la tige de vis (110) ;
lorsque l'élément de commande (240) est dans une seconde position, la structure filetée interne (232) est en prise avec la tige de vis (110) par le biais d'une transmission filetée ;
dans lequel le premier corps de liaison (222) est pourvu d'une partie de liaison (202), et l'appareil de pose comprend en outre un tube de pose (400) pour la pose d'un stent vasculaire, et une extrémité du tube de pose (400) est reliée à demeure à l'élément de liaison (220) par le biais de la partie de liaison (202) ;
dans lequel l'unité mobile (200) comprend en outre un manchon (250), le manchon est manchonné sur l'extérieur de la tige de vis (110), et le manchon (250) est pourvu d'un trou traversant (252), l'élément d'accouplement (230) est disposé sur le manchon (250), et la structure filetée interne (232) peut passer à travers le trou traversant (252) pour être en prise avec la tige filetée (110), l'élément d'accouplement (230) est en outre pourvu d'une protubérance (234) espacée de la structure filetée interne (232) dans la direction circonférentielle, la protubérance (234) est disposée dans une direction opposée à la tige de vis (110), et l'élément de commande (240) est pourvu d'un corps de pression (242) et d'un corps d'extrusion (244) espacé du corps de pression (242) ;
lorsque l'élément de commande (240) est dans la première position, le corps d'extrusion (244) est décalé par rapport à l'élément d'accouplement (230), et le corps de pression (242) est ajusté par pression à la protubérance (234), de telle sorte que la structure filetée interne (232) soit séparée de la tige de vis (110) ; et
lorsque l'élément de commande (240) est dans la seconde position, le corps de pression (242) est décalé par rapport à la protubérance (234), et le corps d'extrusion (244) est ajusté par pression à l'élément d'accouplement (230), de telle sorte que la structure filetée interne (232) soit en prise avec la tige de vis (110) par le biais d'une transmission filetée, et la première poignée (210) peut entraîner la rotation de l'élément de commande (240) et de l'élément d'accouplement (230).

2. Appareil de pose selon la revendication 1, dans lequel l'élément de commande (240) est disposé dans le manchon (250) et peut être remis en place de manière élastique, l'élément de commande (240) peut se remettre automatiquement dans la seconde position sous l'effet d'une force élastique ; et une structure d'introduction de surface inclinée (236) est prévue entre le corps d'extrusion (244) et l'élément d'accouplement (230).

3. Appareil de pose selon la revendication 1, dans lequel l'élément d'accouplement (230) est disposé sur la tige de vis (110) et peut être remis en place élastiquement, l'élément de commande (240) est pourvu d'une partie d'insertion ;
lorsque l'élément de commande (240) est dans la première position, la partie d'insertion est insérée entre la structure filetée interne (232) et la tige de vis (110), de telle sorte que la structure filetée interne (232) soit séparée de la tige de vis (110) ; et
lorsque l'élément de commande (240) est dans la seconde position, la partie d'insertion est décalée à la fois par rapport à la structure filetée interne (232) et à la tige de vis (110), et l'élément d'accouplement (230) est remis en place sous l'effet d'une force élastique, de telle sorte que la structure filetée interne (232) soit en prise avec la tige de vis (110) par le biais d'une transmission filetée.

4. Appareil de pose selon la revendication 1, dans lequel l'unité de fixation (100) comprend en outre une deuxième poignée (120), la deuxième poignée (120) est fixée sur la tige de vis (110), et la première poignée (210) est mobile par rapport à la deuxième poignée (120).

5. Appareil de pose selon la revendication 1, dans lequel le dispositif de commande comprend en outre une unité de flexion (300), l'unité de flexion (300) comprend un élément d'entraînement (310) et un élément de traction flexible (320), l'élément d'entraînement (310) est disposé de manière mobile sur la première poignée (210), et l'élément d'entraînement (310) est mobile avec la première poignée (210), et une extrémité de l'élément de traction flexible (320) est fixée sur l'élément d'entraînement (310).

6. Appareil de pose selon la revendication 5, dans lequel l'élément d'entraînement (310) peut tourner par rapport à la première poignée (210), et l'élément d'entraînement (310) est pourvu d'un manchon de liaison (312) manchonné sur la tige de vis (110) et d'une partie d'application de force (314) fixée sur l'extérieur du manchon de liaison (312) ; une extrémité de l'élément de traction flexible (320) est fixée sur le manchon de liaison (312) ; ou le manchon de liaison (312) est pourvu d'une structure filetée externe (302), et l'unité de flexion (300) comprend en outre une troisième poignée (330) reliée à demeure à la première poignée (210) et un manchon fileté (340) qui est vissé et en prise avec la structure filetée externe (302), la troisième poignée (330) est creuse, le manchon fileté (340) est disposé dans la troisième poignée (330) et est relié de manière coulissante à la troisième poignée (330), une extrémité de l'élément de traction flexible (320) est fixée sur le manchon fileté (340).

7. Appareil de pose selon l'une quelconque des revendications 1 à 6, dans lequel l'unité de fixation (100) comprend en outre une tige de guidage (130), la tige de guidage (130) est fixée dans la tige de vis (110), et le premier corps de liaison (222) est pourvu d'un trou d'accouplement (204) qui est ajusté par glissement à la tige de guidage (130).

8. Appareil de pose selon la revendication 7, dans lequel la tige de guidage (130) est pourvue d'un canal d'écoulement, et l'unité de fixation (100) comprend en outre un tube de guidage (140) en communication avec le canal d'écoulement, et une extrémité du tube de guidage (140) est fixée à la tige de vis (110) et communique avec l'extérieur.
